Europäisches Patentamt

European Patent Office

Office européen des brevets

(11) Publication number: **0 309 111**
**A2**

# (12) EUROPEAN PATENT APPLICATION

(21) Application number: 88308168.9

(22) Date of filing: 02.09.88

(51) Int. Cl.4: **C12N 9/64** , **C12N 15/00** ,
**A61K 37/54** , **C12N 5/00**

(30) Priority: 03.09.87 US 92502
29.08.88 US 236590

(43) Date of publication of application:
29.03.89 Bulletin 89/13

(84) Designated Contracting States:
AT BE CH DE ES FR GB GR IT LI LU NL SE

(71) Applicant: INVITRON CORPORATION
4649 Le Bourget Drive
St. Louis Missouri 63134(US)

(72) Inventor: Prior, Christopher P.
1506 Hawk Forest Road
Ballwin Missouri 63021(US)
Inventor: Hope, James
601 Amberwood Lane
Manchester Missouri 63011(US)
Inventor: Tolbert, William R.
1415 Monttrore Drive
Manchester Missouri 63011(US)

(74) Representative: Goldin, Douglas Michael et al
J.A. KEMP & CO. 14, South Square Gray's Inn
London WC1R 5EU(GB)

(54) **A process for obtaining higher yields of unaggregated tissue plasminogen activator from cell culture.**

(57) Aggregation of tPA during cell culture production, and during the period prior to purification, results in significantly decreased yields of the purified monomeric form. Aggregated tPA has a substantially lower specific activity than the monomeric form, and is potentially immunogenic. The aggregated form results from molecular changes which occur during the incubation under cell culture conditions, i.e., at temperatures of around 37° C in conditioned medium. The problem of aggregation of tPA is overcome by removing tPA containing effluent from the cell culture conditions while substantially more unaggregated (monomeric) tPA is present than aggregated tPA, and maintaining the effluent under conditions which prevent the aggregation, for example, temperatures around 4° C. The use of perfusion systems for the cell culture production of tPA also overcomes the aggregation problem because of diminished exposure of the tPA containing effluent to cell culture conditions, and the ease with which the effluent can be transferred to desirable conditions which lessen or prevent aggregation.

EP 0 309 111 A2

# A PROCESS FOR OBTAINING HIGHER YIELDS OF UNAGGREGATED TISSUE PLASMINOGEN ACTIVATOR FROM CELL CULTURE

## Technical Field

The invention relates to methodologies for preparing human tissue plasminogen activator (tPA) on a scale which is commercially useful. More specifically, it relates to methods for obtaining higher yields of active tPA from cells in culture.

## References

Abrams. P.G. et al (1984), J. Immunol. 132. 1611.
Feder. J. and Tolbert, W.R. (1983), Am. Biotech. Lab. 3, 24.
Fiechter. A. et al (1987) in THE YEASTS vol. 2. 2nd edition (Rose. A.H. and Harrison. J.S., eds, Academic Press, London) pp. 99-129.
Friberger, P. et al (1979), Prog. in Chem. Fibr. & Thromb. IV (Davidson, J.A.F., ed), p. 149.
Friberger, P. (1982), Thesis Scand. J. Clin. Lab Invest. 42 Suppl. 162, p.58.
Kaufman, R.J. and Sharp, P.A. (1982), J. Mol. Biol. 159, 601.
Klausner, A. (1986), Biotechnology 4, 706.
Laemmli. U.K. (1970), Nature 227. 680.
Ny et al (1984), Proc. Natl. Acad. Sci, USA 81, 5355
Pennica et al (1983). Nature, 301. 214.
Reuveny et al (1986), J. Immunol. Methods 86, 61.
Thorsen (1975), Biochim. Biophys. Acta 393, 55.
Tolbert et al (1980), Ann. Biochem. 106, 109.
Tolbert et al (1985), LARGE-SCALE MAMMALIAN CELL CULTURE (Feder. J. and Tolbert. W.R., eds, Academic Press. Inc.) p97.
Tolbert et al (1987) ANIMAL CELL BIOTECHNOLOGY (Griffiths. G.B., ed. Academic Press. London. in press).
Van Brunt (1986), Biotechnology 4. 505.
van Wezel, A.L. (1967), Nature 216. 64.
UK patent GB 2,119.804B.
U.S. Patent Number 4,661,453.
U.S. Patent Application Serial Number 010.871.
U.S. Patent Number 4,166.768.
U.S. Patent Number 4,178,209.
U.S. Patent Number 4,184.916.
U.S. Patent Number 4,537,860.
U.S. Patent Number 4,335,215.
European Patent Application 41,766.
European Patent Application Number 85.306432.7.

## Background Art

Human tissue plasminogen activator (tPA) is a serine protease that converts plasminogen into plasmin. Plasmin degrades the fibrin matrix of blood clots. thereby restoring the hemodynamic condition of an open vascular system after an internal vascular accident has produced thrombosis or thromboembolism. Vascular disease states involving partial or total blockage of blood vessels which are amenable to treatment with plasminogen activators include stroke, pulmonary embolism, myocardial infarction. as well as deep vein and peripheral artery obstructions.

tPA is one of two immunologically distinct types of plasminogen activators found in human plasma and other body fluids. It has an $M_r$ of 68,000. and its activity is potentiated by fibrin. The enzyme acts at the site of a thrombus, and demonstrates a higher affinity for fibrin than does the other plasminogen activator, i.e., the urokinase-type plasminogen activator. Thus, tPA is considered to be the physiologically relevant thrombolytic agent and is considered to have significant potential as a therapeutic agent.

Due to the extremely low concentration of tPA in blood and tissue extracts, other sources and means of producing this preferred thrombolytic agent have been sought after. Preferred methods of production for commercial utilization involve growing cells capable of producing tPA in culture, including cells transformed with recombinant vectors which encode this protein. A preferred method for producing tPA, including that expressed from recombinant DNA vectors, is in mammalian cell culture systems. Mammalian cells have the capability to synthesize human proteins with the proper configuration, correct disulfide bonding, and arrays of sugar side chains which all result in a protein which mimics the naturally occurring protein in terms of activity and lack of immunogenicity.

A DNA sequence encoding tPA and the amino acid sequence of tPA is disclosed by Pennica et al (1983), Ny et al (1984), and UK patent GB 2,119,804 B. tPA has been synthesized in a variety of cells, including those transformed with recombinant expression vectors containing sequences which encode tPA and its derivative analogs. See, for example, Van Brunt (1986).

A great diversity of procedures and apparatus for in vitro cell culture production of biomolecules of interest has been utilized heretofore. Generally, the procedures for cell culture utilizing the different apparatus may be classified as batch procedures, semi-batch procedures, and continuous or perfusion procedures. In batch procedures the cells are grown in a fixed amount of nutrient and harvested after significant growth ceases; the desired product is isolated from the cell culture after the first round of growth. Semi-batch procedures are similar to batch procedures; however, after harvest the cells are submitted to a repeated round(s) of growth and expression by resuspension in fresh medium. In perfusion processes the cells are maintained in the culture vessel, and fresh medium is perfused in while spent medium (also called conditioned medium) is withdrawn in a continuous or semi-continuous process. Cell propagation processes have been reviewed in Van Brunt (1986), Reuveny et al (1986), and Feder, J. and Tolbert, W.R. (1985), and Tolbert et al (1985).

## Disclosure of the Invention

The invention results from the discovery that batch processes, including semi-batch processes, for the production of tPA by cells in culture yield in large part tPA in an aggregated relatively inactive form, which results in significantly lower yields of the active product after purification. The formation of aggregated tPA occurs during the continued incubation of the newly synthesized polypeptide under the conditions in which cells expressing tPA are propagated or maintained.

Accordingly, one embodiment of the invention is a process for obtaining higher yields of active tPA from tpA-expressing cells in culture comprising: a) removing the tpA-containing effluent from a cell culture chamber containing tpA-expressing cells while substantially more active unaggregated tPA is present than inactive aggregated tPA; and b) subjecting the removed tPA containing effluent to a condition which prevents said unaggregated tPA from aggregating.

Another embodiment of the invention is a process for obtaining higher yields of active tPA from cells in culture comprising: a) propagating cells capable of producing and secreting tPA in a perfusion system under conditions which allow the cells to synthesize tPA; b) removing tPA containing effluent from the perfusion system while substantially more active unaggregated tPA is present than inactive aggregated tPA; and c) subjecting the removed tPA containing effluent to a condition which prevents said active unaggregated tPA from aggregating.

Yet another embodiment of the invention is a process for obtaining higher yields of unaggregated tissue plasminogen activator (tPA) from tPA expressing cells in culture comprising: a) continuously removing conditioned media from cells producing tPA to a low temperature environment of about 4° C; b) adjusting the pH and ionic strength of the medium to conditions wherein tPA is selectively bound to a cation exchange support; c) treating the adjusted medium with a cation exchange support so that tPA is selectively adsorbed; d) eluting the adsorbed tPA from the cation exchange support; e) adjusting the pH and ionic strength of the tPA containing eluate to conditions wherein tPA is selectively adsorbed to an affinity support; f) treating the adjusted eluate of e) with an affinity support to selectively adsorb the tPA; g) eluting the selectively adsorbed tPA from the affinity support; h) causing the tPA in the tPA containing eluate of g) to precipitate by the addition of salt, and capturing the tPA containing precipitate; i) dissolving the tPA containing precipitate of h) in a solution containing arginine HCl and sodium citrate, wherein the arginine HCl is preferably about 150 mM and the sodium citrate is preferably about 50 mM, and the pH is preferably about 6; j) treating the tPA containing solution of i) with a gel filtration support equilibrated against the solution of i); and k) eluting the tPA from the gel filtration support and recovering the tPA containing fraction.

Brief Description of the Drawings

Figure 1 is an illustration of a perfusion chemostat system.

Figure 2 is an illustration of a microcarrier reactor perfusion system.

Figure 3 is an illustration of a static maintenance reactor perfusion system.

Figure 4 is a graph showing the growth of CHO cells expressing tPA when the product is produced by the perfusion process.

Figure 5 is a graph showing the growth of CHO cells expressing tPA when the product is produced by the semi-batch process.

Figure 6 is a graph showing the growth of CHO cells expressing tPA when the product is produced by the batch process.

Figure 7 is a graph showing the tPA containing peaks eluted from gel filtration chromatography.

Figure 8 is a photograph showing the analysis by gel electrophoresis of the tPA containing fractions obtained from gel filtration.

Figure 9 is a photograph showing the analysis by gel electrophoresis under reducing and non-reducing conditions of tPA species of about 70,000 molecular weight and about or greater than 110.000 molecular weight, isolated by gel filtration.

Figure 10 is a graph showing the time course of inactivation of tPA in conditioned medium containing serum, when maintained at 37° C compared to 4° C, using the S-2251 assay.

Figure 11 is a graph showing the time course of inactivation of tPA in conditioned medium without serum, when maintained at 37° C compared to 4° C, using the S-2251 assay.

Figure 12 is a graph showing the time course of inactivation of tPA in conditioned medium containing serum, when maintained at 37° C compared to 4° C, using the S-2288 assay.

Figure 13 is a graph showing the time course of inactivation of tPA in conditioned medium without serum, when maintained at 37° C compared to 4° C, using the S-2288 assay.

Modes for Carrying Out the Invention

A. Definitions

As used herein, the term propagating cells in a "perfusion system" is defined as a method of cell culture in which the cells are held in a growth chamber or vessel, while growth medium is passed or recirculated around the cells. There is a continuous or semi-continuous addition of medium containing nutrients to the cells, and continuous or semi-continuous removal of conditioned medium from the cells.

"Conditioned medium" is defined as nutrient medium for cell culture which has been exposed to the cells under culture conditions, and which contains cellular products resulting from cellular metabolism and synthesis.

"Control sequence" refers to DNA sequences which are necessary to effect the expression of coding sequences to which they are ligated. The nature of such control sequences differs depending on the host organism; in eukaryotes, generally, such control sequences include promoters, terminators, and in some instances, enhancers. The term "control sequences: is intended to include, at a minimum, all components whose presence is necessary for expression, and may also include additional components whose presence is advantageous to the expression of the desired gene.

"Operably linked" refers to a juxtaposition wherein the components are in a relationship permitting them to function in their intended manner. A control sequence "operably linked" to a coding sequence is ligated in such a way that expression of the coding sequence is achieved under conditions compatible with the control sequences.

"Expression system" refers to a DNA segment which contains at least one "expression cassette", which cassette contains a coding sequence to be expressed and the control sequences, operably linked to it, which enable its expression.

"Effluent" refers to cell culture medium containing tPA synthesized by cells contained in a cell culture vessel. The terms "effluent" and "conditioned medium" are used interchangeably herein.

As used herein, the term "tissue plasminogen activator" (tPA) is meant to include both the single chain and double chain species of the enzymes encoded in wild-type tPA genes including variants of wild-type tPA genes, as well as polypeptides encoded within DNA sequences derived from a wild-type gene by mutation. Mutations may be induced in the wild type gene by known mutagenic techniques, including those involving recombinant DNA techniques. "Wild-type tPA" refers to tPA structures which occur naturally. See Klausner (1986) for a review of examples of tPA species under current investigation.

The term "non-aggregated tPA" refers to tPA which is in the native single chain or double chain conformation. Human non-aggregated tPA when subjected to gel filtration migrates with a molecular weight of about 70,000 daltons. This material, when subjected to polyacrylamide gel electrophoresis in sodium dodecyl sulfate under the conditions described by Laemmli (1970), in the absence of reducing agents migrates with a molecular weight of approximately 70,000 daltons. Using analogous conditions, but adding reducing agents yields a migration pattern which is dependent upon the relative amounts of single and double chain present. The single chain migrates with an apparent molecular weight of about 70,000 daltons, and the dissociated A and B chains of the two chain species migrate in close proximity with an apparent molecular weight of about 30,000 daltons. The terms "non-aggregated" and "monomeric" are used interchangeably herein with respect to tPA.

"Aggregated tPA" refers to tPA which is a larger molecular weight species due to aggregation of two or more monomeric molecules with each other, or with protein(s) present in the cell culture medium. For example, aggregated human tPA when subjected to gel filtration or polyacrylamide gel electrophoresis on Laemmli gels under non-reducing conditions migrates with an apparent molecular weight of about 110,000 or greater.

The term "inactive" as used with respect to aggregated tPA refers to the substantially lowered specific enzymic activity of the aggregated form relative to that of the unaggregated form.


## B. General Description

The practice of the present invention employs, unless otherwise indicated, conventional techniques of cell culture, molecular biology, microbiology, recombinant DNA, and immunology, which are within the skill of the art. Such techniques are explained fully in the literature. See, e.g., ANIMAL CELL CULTURE (R.K. Freshney, ed 1986); Maniatis, Fritsch and Sambrook, MOLECULAR CLONING: A LABORATORY MANUAL (1982); DNA CLONING, Volumes I and II (D.N. Glover ed 1985); OLIGONUCLEOTIDE SYNTHESIS (M.J. Gait ed 1984); NUCLEIC ACID HYBRIDIZATION (B.D. Hames and S.J. Higgins, eds 1984); B. Perbal, A PRACTICAL GUIDE TO MOLECULAR CLONING (1984); the series, METHODS IN ENZYMOLOGY (Academic Press, Inc.), GENE TRANSFER VECTORS FOR MAMMALIAN CELLS (1987) J.H. Miller, and M.P. Calos, eds, Cold Spring Harbor Laboratory), LARGE-SCALE MAMMALIAN CELL CULTURE (Feder, J. and Tolbert, W.R., eds, Academic Press, Inc., N.Y.), and ANIMAL CELL BIOTECHNOLOGY (Griffiths, G.B., ed, Academic Press, London, in press).

All patents and publications mentioned herein, both supra and infra, are hereby incorporated by reference.

In the invention, the types of cells used are any cells which are capable of producing tPA at levels suitable for commercial production, i.e., preferably at least about 1 mg tPA / liter packed cells / day, and which secrete the product into the cell culture medium. A variety of cells which are capable of expressing tPA are known in the art. For example, rat prostate adenocarcinoma cells , U.S. Patent number 4,661,453, and a human melanoma cell line (Bowes) (European Patent Application 41,766, published Dec. 16, 1981; Rijken and Collen (1981), and Kluft et al (1983)).

The cells expressing tPA may also be cells transformed with recombinant vectors containing a nucleotide sequence encoding tPA operably linked to control sequences which allow its expression and secretion. Transformed cells of this type include bacterial cells, for example, transformed E. coli cells, transformed B. subtilis cells, and the like. Also included are fungal cells, for example, yeast cells. See, for example, European Patent Application Number 85306432.7. Mammalian cells may also be transformed with a recombinant vector which contains an expression system which encodes tPA. For example, as discussed below, the synthesis of tPA may be by Chinese hamster ovary cells (CHO) transformed with a vector pSTH-MDH, which contains the tPA sequences and an amplifiable DHFR gene. The construction of pSTH-MDH and the CHO host cell line are described in commonly owned U.S. patent application serial number 010,871; examples of other vectors which may also be used to transform mammalian cell lines are also described in this application, and include pSV-tPA17, and pSTH-SDH. Another vector which may be used to transform CHO cells is that described by Kaufman and Sharp (1982).

The tPA expressing cells are propagated or maintained in a culture system which allows for the removal of the effluent when the ratio of active unaggregated tPA to aggregated inactive tPA is present in a desired range. Batch, semi-batch, or perfusion culture systems may be used, and protocols for these systems and their design are known in the art. See, for example, Fiechter et al (1987), Tolbert et al (1987), and Tolbert et al (1985). The cell culture conditions are those which maximize tPA production and cell viability, and are dependent upon the cell-types employed, as well as the type of culture system employed. Cell culture conditions include cell culture media, proportions of gases, temperature, pH and other parameters known in the art. The desired conditions may be established by routine experimentation.

Generally, in batch techniques, which include semi-batch techniques, cell culture reactors having a volume of 1000 to 10,000 liters are charged at the beginning of the production cycle with seed culture of cells at a density of a few tenths of a milliliter per liter and sufficient medium to sustain the anticipated cell mass during the entire production cycle. As the cell culture grows, the cells utilize the nutrients and produce desired proteins and waste products, both of which remain in the vessel. Under these conditions, most cultured cells are limited in population density, and with animal cells, the limit is approximately one to two milliliters of cells per liter of reactor liquid volume. In batch culture the cells usually are grown to maximum cell density, which with mammalian cells generally occurs anywhere from four to twenty days after the process begins, depending on the cell line's growth characteristics. The desired product may be synthesized during the phase of logarithmic growth and/or during stationary phase. After the desired amount of synthesis is achieved the contents of the reactor are then processed to isolate the tPA from the cells, waste products, and remaining nutrients.

In perfusion culture, medium is perfused through the reactor at a rate proportional to the number of cells and their metabolic characteristics. Process parameters can be maintained by introducing reagents and gases directly into the vessel or into the medium as it is being fed to the vessel. Effluent is removed from the perfusion chamber on a continual or semi-continuous basis. Examples of perfusion systems are described in Tolbert et al (1985), in Tolbert et al (1987), and component parts in U.S. Patent Numbers: 4,166,768; 4,178,209; 4,184,916; 4,537,860; and 4,335,215.

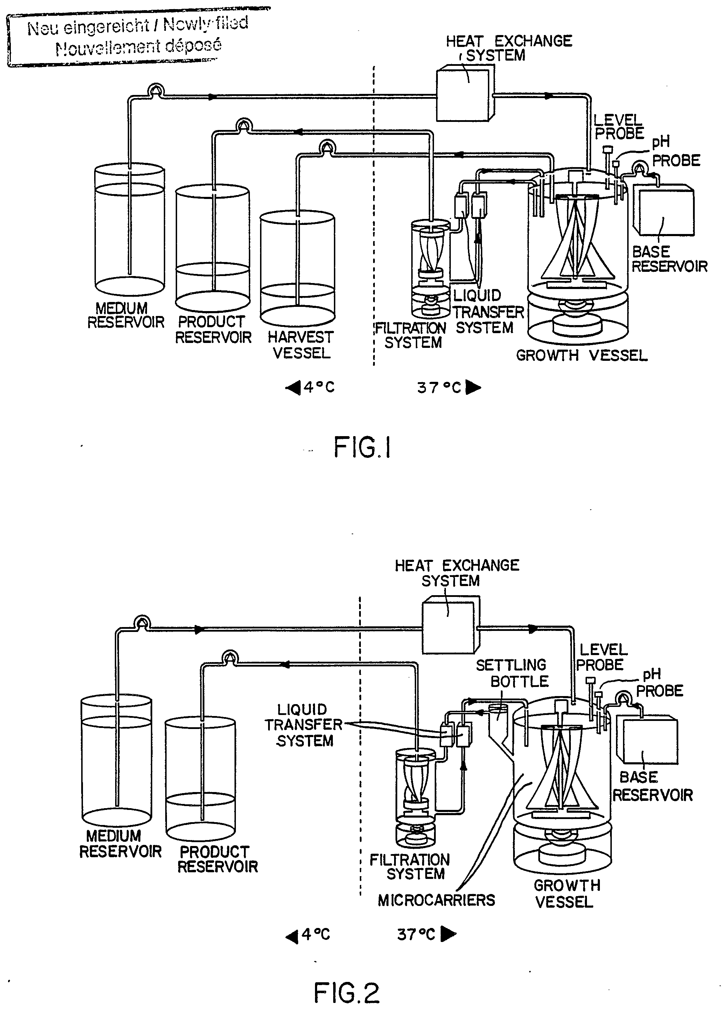

Mammalian cells which grow in suspension culture may be used to produce tPA in a perfusion culture system, an example of which is shown in figure 1 and parts of which are described in U.S. Patent Numbers 4,166,768; 4,178,209; and 4,184,916. In this system a constant environment is maintained in the growth vessel by continually adding fresh nutrients and removing waste products at a rate proportional to the number of cells in the vessel. Under these conditions, the system can operate for prolonged periods of time with cell densities from 10 to 30 times the maximum cell density in a typical batch reactor. A gentle "sail" agitator system is used to prevent cell damage at these high cell densities. The filtration unit separates the cells from the product containing cell conditioned medium and returns the cells to the growth vessel as fresh medium is added. Dissolved oxygen and carbon dioxide, as well as pH levels, are continually monitored and maintained in present ranges throughout the production run. The effluent from the reactor contains waste products and the desired protein products secreted by the cells. Product reservoirs may be removed periodically for further processing by concentration and other conventional protein purification techniques.

When prolonged cell growth is required for production of desired protein products, the suspension culture perfusion system maintains an optimal cell density in the growth vessel by transferring excess cells to the harvest vessel. After the optimal cell density has been attained in the growth vessel, the serum concentration in the fresh medium can be significantly reduced.

In the Examples, tPA is expressed by CHO cells in suspension perfusion culture expressing the tPA sequence in the vector, pSTH-MDH. Using this system, an optimal range of synthesis of tPA may be obtained when the packed cell volume (PCV) is in the range of about 10 ml to about 14 ml per liter of cell culture.

Many mammalian cells require attachment to a surface for growth, optimal production of proteins or for survival. For such cases a microcarrier method may be used with, for example, a reactor similar to the one described in figure 2, which is described in U.S. Patent Number 4,335,215. Examples of microcarrier methods are known in the art, see for example, the one described by van Wezel (1967). The microcarrier reactor system requires very gentle agitation. By providing the necessary agitation energy through as large a surface area as possible with an agitator rotating between 8 and 12 rpm, much higher amounts of microcarriers, and therefore surface area, may be used. Up to one fourth the settled volume of the reactor can be microcarriers.

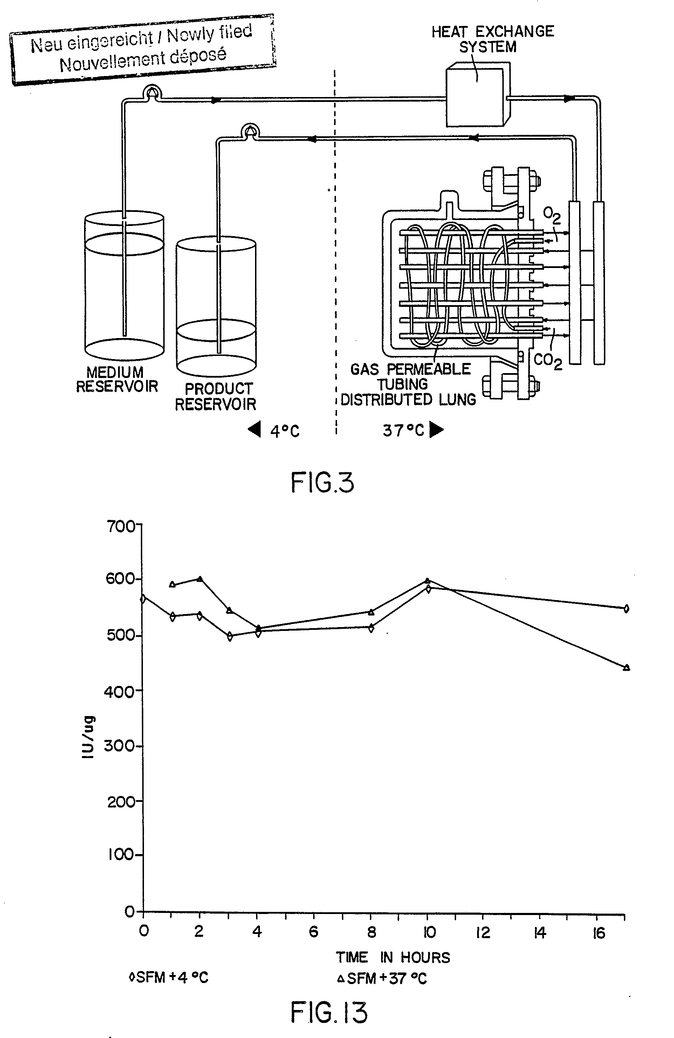

Another perfusion culture system which may be used when it is desirable to maintain tPA expressing cells in a non-proliferative state is one which utilizes a static maintenance reactor, an example of which is illustrated in figure 3, and which is described in U.S. Patent Number 4,537,860. In this system one to two kilograms of cells are grown in either of the above described perfusion growth systems (as illustrated in figures 1 and 2) and then concentrated, mixed with a finely divided matrix material and then pumped as a thick slurry into the reactor. The reactor is filled until the cells are completely immobilized in the interstices of the matrix material. About nine tenths of the reactor volume is matrix, and about one tenth is evenly distributed cell mass. The static maintenance reactor may be used to maintain both anchorage-dependent cells and suspension cells at densities approximately 100 times that obtained in a conventional batch system.

In the example shown in figure 3 the static maintenance reactor consists of a cylindrical vessel penetrated by an array of porous tubes. One set of tubes allows perfusion of a fresh nutrient medium into the reactor and the second set of adjacent tubes removes cell conditioned medium containing product. All cells are within about two centimeters of a fresh source of nutrient medium. A distributed semi-permeable membrane is also provided to allow diffusion of oxygen to the cells and diffusion out of carbon dioxide.

Perfusion culture systems are preferable to batch culture methods since generally they allow for control over the perfusion rate, more convenient periodic or continuous removal of the effluent as well as the achievement of higher cell densities which have the potential of yielding greater amounts of tPA.

As shown in the Examples, prolonged incubation of tPA in conditioned cell culture medium under conditions of cell growth or expression, leads to significant aggregation of the tPA product. The aggregated products are enzymically much less active than the unaggregated product, and in addition pose an immunogenic threat if co-purified as trace contaminants with the final tPA product. Therefore, in batch systems the synthetic reaction is halted when the ratio of unaggregated tPA to aggregated tPA is in the desired ratio, which is preferably at least 2:1, is more preferably at least 3:1, and even more preferably is at least 4:1. The time at which synthesis is stopped depends upon both the amount of tPA synthesized and the ratio of unaggregated to aggregated tPA. When synthesis is stopped, the contents of the reactor are subjected to conditions which lessen the aggregation of unaggregated tPA. For example, this may be achieved by rapidly chilling the reaction mixture to a temperature at which the aggregation is largely prevented. These temperatures are usually in the range of about 0°C to about 15°C, are preferably in the range of about 1°C to about 10°C, and even more preferably are in the range of about 2°C to about 6°C. It may also be accomplished, for example, by beginning the isolation of tPA from the remainder of the components of the cell culture medium. The tPA can then be isolated and purified by known conventional techniques. In order to produce additional product, the cyclical growing process is reinitiated.

Aggregation of tPA during commercial production may also be overcome by producing it in systems which use perfusion bioreactors. As shown in the Examples, in cases where cell densities were held comparable in batch, semi-batch, and perfusion modes, the shorter product residency time in the perfusion vessel (hours) compared to semi-batch (days/weeks) and batch (weeks) yields substantially less aggregated tPA in the effluent, resulting in a higher specific activity of tPA in this crude starting material, and an increased product yield after purification.

In addition to the short residency in the vessel, perfusion systems also allow for the expeditious change of conditions for the product in the effluent after it is removed from the vessel. Thus, product can be maintained under conditions which significantly retard or prevent the molecular changes associated with its aggregation. The changed conditions may include, for example, change in temperature and/or the beginning of purification of the product, as described for the Batch processes.

The amounts and ratios of unaggregated and aggregated tPA in the effluent may be determined by any method which conveniently distinguishes the species, for example, by the difference in molecular weight on gel filtration or upon gel electrophoresis using Laemmli gels under non-reducing conditions. The presence of tPA in the fractions obtained by molecular weight sizing materials can be detected by procedures known in the art, for example, by determination of the enzymic tPA activity, or by immunoassay. An Elisa assay for tPA is described in Abrams et al (1984), and kits providing the reagents for Elisa assays of tPA are commercially available.

Additionally, the ratio of unaggregated and aggregated tPA in the effluent can be estimated by, for example, the specific enzymic activity of the tPA in the effluent, i.e., the enzymic activity in I.U./mg tPA. Monomeric tPA has an expected specific activity of about 440,000/mg tPA; aggregated tPA displays residual enzymic activity, however, for purposes of the calculation it may be assumed that the activity of the aggregated species is negligible.

In vivo, and usually in vitro, wild-type monomeric tPA is synthesized as a single chain proenzyme of approximate molecular weight of 70,000, which can be cleaved by plasmin or trypsin, without disrupting the disulfide linked two-chain molecular structure. Upon reduction, tPA dissociates into a heavy and a light chain, with approximate molecular weights of 35,000. Wild type tPA contains two kringles linked by a hexapeptide linker sequence. A kringle is a triple disulfide-linked sequence of amino acids which form a loop. These kringles are believed to be responsible for binding of tPA to fibrin. Thorsen (1975).

Incubation of monomeric tPA under cell culture conditions induces the formation of aggregated tPA from monomeric tPA. See Examples C.6. and C.7., where it can be seen that monomeric human tPA which has an apparent molecular weight of approximately 70,000 when it is subjected to gel chromatography or to polyacrylamide gel electrophoresis upon Laemmli gels under non-reducing conditions, is converted to a species with an apparent molecular weight of approximately 110,000 or greater by incubation in the presence of conditioned medium. As shown in Example C.7., the conversion occurs with time, and at 96 hours at 37°C approximately half of the original monomeric species has been converted to the aggregated form. The conversion of monomeric species to the aggregated form is also manifested by a lowered specific enzymic activity of the tPA.

Aggregation may involve at least two separate and perhaps interrelated phenomena. Part of the change in molecular weight may be due to the association of tPA with -antiplasmin and proteinase inhibitors present in serum used in the cell culture medium. The tpA-inhibitor complex is not dissociated by boiling in the presence of reductant and SDS, thus, the interaction may be covalent. Another cause of aggregation may be the formation of intermolecular disulfide bonds, as evidenced by the finding that a portion of the 110,000 dalton species migrates with apparent molecular weights of approximately 30,000 daltons when subjected to electrophoresis on Laemmli gels in the presence of a reducing agent.

Information derived from routine experiments in which the conversion of the monomeric, i.e., unaggregated species of tPA, to the aggregated species is monitored as a function of time and incubation conditions is useful in calculating when the effluent should be removed from the culture vessel, since it gives an approximate expected ratio of the two species. It is also useful in determining the conditions to which the removed effluent should be subjected to prevent or reduce further aggregation. An example of this type of experiment is in Sections C.7. through C.12., inclusive.

The tPA in the removed effluent may be further purified using processes for protein purification which are known in the art, and which include, for example, ion exchange, affinity, and gel filtration chromatography, centrifugation, and dialysis. As shown in the Examples, the relative amounts of aggregated and unaggregated tPA in the effluent influences greatly the yield of active monomeric tPA isolated by the purification procedure. As shown in the Examples, also, when the perfusion technique is compared to the batch and semi-batch techniques for the synthesis of tPA from transformed CHO cells, effluents from the perfusion system contain tPA of higher specific activity, which is a reflection of the increased proportion of monomeric tPA relative to aggregated tPA. This increased proportion of monomeric tPA is also reflected in the increased yields of the active monomeric species of the tPA after purification. Thus, the invention describes processes which yield substantially higher proportions of monomeric tPA as starting material for purification, which in turn results in substantially higher yields of the desired active monomeric species after purification.

The tPA produced by the processes of the invention, when purified, may be used for the purposes already described for tPA, including therapy for disorders related to blood clots. In such cases the product will be formulated in pharmacologically acceptable compositions, and administered in therapeutically acceptable doses. In addition, the tPA produced by the invention is useful as a standard which may be used, for example, for monitoring isolation techniques for tPA. The product is also useful for diagnostic techniques which monitor disorders related to disfunctions in the level of tPA.

## C. Examples

The following examples are intended to illustrate but not to limit the invention.

## C.1. General Methods

Polyacrylamide gel electrophoresis is carried out as described by Laemmli (1970) in the presence or absence of sulfhydryl reducing reagents.

Analysis of tPA enzymic activity is performed using a direct amidolytic synthetic substrate assay, essentially as described by Friberger et al (1979) and Friberger (1982) in the presence of plasmin and soybean trypsin inhibitor, and using as substrate S-2288.

Immunoblotting techniques and Elisa assays for the detection and/or quantitation of tPA are performed as described by Abrams (1984), using as antibody a mouse IgG[1] monoclonal antibody which recognizes an epitope in the A chain of human tPA.

Cell density is determined as packed cell volume (PCV) as described by Tolbert, W.R. et al (1980).

### C.2. Cell Culture Using Batch, Semi-Batch, and Perfusion Techniques

CHO cells transformed with the vector pSTH-MDH, as described in commonly owned U.S. Patent application serial number 010,871, are grown in Hamm's S12 - GHT cell growth medium containing 7.5% Fetal Calf Serum and methotrexate.

### C.2.a. Growth in a Perfusion System

In case I, the cells are grown in a perfusion system as illustrated in figure 1, and the rate of the perfusion is balanced to the PCV in an effort to deliver nutrients and remove waste products at a rate compatible with cell metabolism. Under these conditions, which are analogous to an in vivo environment, the cells can grow to at least a 10 fold higher PCV, i.e., about 10-14 ml/L, resulting in substantially elevated production of tPA levels relative to those achievable by batch or semi-batch procedures. However, to compare the results obtained with the three cell culture techniques, the perfused media is collected at a cell density comparable to those achievable with batch and semi-batch procedures over a 7 day period, i.e., PCV 0.4 ml/L - 1.6 ml/L. The cells are maintained in logarithmic growth during the period in a 100 liter perfusion reactor, and the effluent is immediately transferred to 4° C during collection.

### C.2.b. Growth in a Semi-Batch System

In case II, which is a semi-batch process, cells are grown logarithmically and allowed to reach the optimal PCV of 0.9 ml/L-1.0 ml/L at the 5th day, at which time the fluid in the 14 liter reactor is changed for fresh cell culture medium so that logarithmic growth can continue; the fresh medium represents approximately 70% of the reactor volume. At the time the medium is changed, the cell density drops to PCV 0.19 ml/L, and recovers to PCV of 0.9 ml/L after the cells have grown logarithmically for 11 days. The 5 day effluent is stored at 4° C, and the two fluid changes are pooled at the eleventh day. In this process cells are not allowed to deteriorate, however, they are subjected to a "feast or famine" regimen, which may not be ideal for long term viability and expression.

### C.2.b. Growth in a Batch System

In case III, which is a batch process, the cells are maintained in media for 11 days without any fluid changes. Cells maintained in this "static" batch process reach a typical maximum PCV of 0.9 ml/L - 1.0 ml/L and then begin to deteriorate as the nutrients are depleted and waste products accumulate.

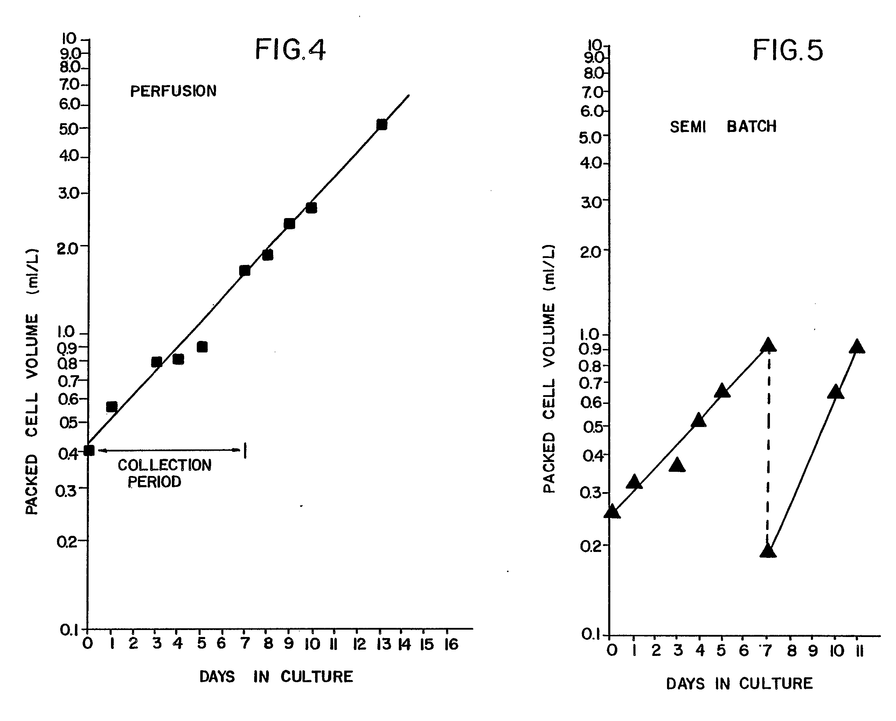

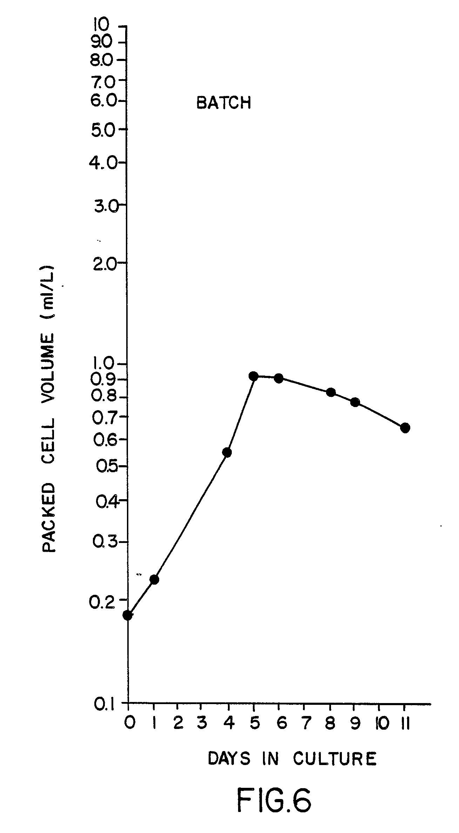

The growth curves of the cells grown in the perfusion system, the semi-batch system, and the batch system are shown in figures 4, 5, and 6, respectively.

### C.3. Comparison of Yields and Enzymic Activity of tPA Isolated From the Effluents of Transformed CHO Cells Propagated in Perfusion, Semi-Batch and Batch Systems

The effluents obtained from Cases I, II, and III described in Section C.2. are subjected to identical purification procedures. The purification process selects for tPA molecules in the monomeric state, and involves ion exchange, affinity, and gel filtration chromatography. Each fraction is analyzed to determine the quantity and specific enzymic activity of tPA. Table 1 summarizes the results obtained during the purification process.

As seen in table 1, the specific activity of tPA in the effluent obtained from the perfusion system (Case I) is substantially higher than that obtained from either the semi-batch (case II) or batch (case III) processes. This difference in specific activity is maintained through chromatography on ion-exchange and affinity columns. After gel filtration, which separates the monomeric tPA from the aggregated species, the specific activities of the tPA in the monomeric fraction isolated from the three effluents are comparable. However, the yield of the high specific activity monomeric tPA is substantially greater from the perfusion system than it is from the batch or semi-batch processes.

Table 1

| Purification of Human rtPA | | | | | | | | | |
|---|---|---|---|---|---|---|---|---|---|
| Step | % Recovery by Antigen | | | % Recovery by Activity | | | Specific Activity (IU/mg tPA) | | |
| | III Batch | II Semi-Batch | I Perfusion | III Batch | II Semi-Batch | I Perfusion | III Batch | II Semi-Batch | I Perfusion |
| Conditioned Media (Start) | 100 | 100 | 100 | 100 | 100 | 100 | 74,100 | 224,900 | 319,800 |
| Ion Exchange | 95 | 95 | 95 | 100 | 100 | 93 | 76,700 | 246,700 | 279,600 |
| Affinity | 65 | 61 | 70 | 92 | 100 | 85 | 114,400 | 391,300 | 392,600 |
| Gel Filtration (Final Purified) | 6 | 21 | 65 | 47 | 45 | 100 | 447,200 | 492,700 | 483,600 |

EP 0 309 111 A2

The relative production of purified tPA derived from the three culture conditions is summarized in Table 2.

Table 2

| | III Batch | II Semi-Batch | I Perfusion |
|---|---|---|---|
| Purif. Yield | 8% | 21% | 65% |
| Relative Production of Purified Material | 1% | 1.33% | 7.74%* |

* Cell density comparable to II and III.

In Table 2 the values are normalized on the basis of mg purified tpA/liter/day. As seen from Table 2, cells propagated in the perfusion system yield approximately 6-7 fold more purified tPA than do those from the batch or semi-batch systems. It should be noted, however, that the value obtained with the perfusion system is an undervaluation of what can be obtained from this system. The pooled effluents used to isolate the tPA in Case I are from periods in which the PCV is much lower than that which would normally be used, i.e., the collection is from a period which spans a PCV of 0.4 to 1.6. Under normal conditions cell densities of a PCV of greater than 10 are achieved.

C.4. Characterization of the Species of tPA Obtained from Perfusion, Batch, and Semi-Batch Processes by Gel Filtration Chromatography

The final gel filtration step of the procedure in Section C.3. separates tPA species on the basis of size. tPA elutes from the gel filtration column as essentially two molecular weight species, i.e., monomeric tPA migrates as having an apparent molecular weight of approximately 70,000 daltons, and aggregated tPA migrates as having an apparent molecular weight of approximately 110,000 daltons or greater.

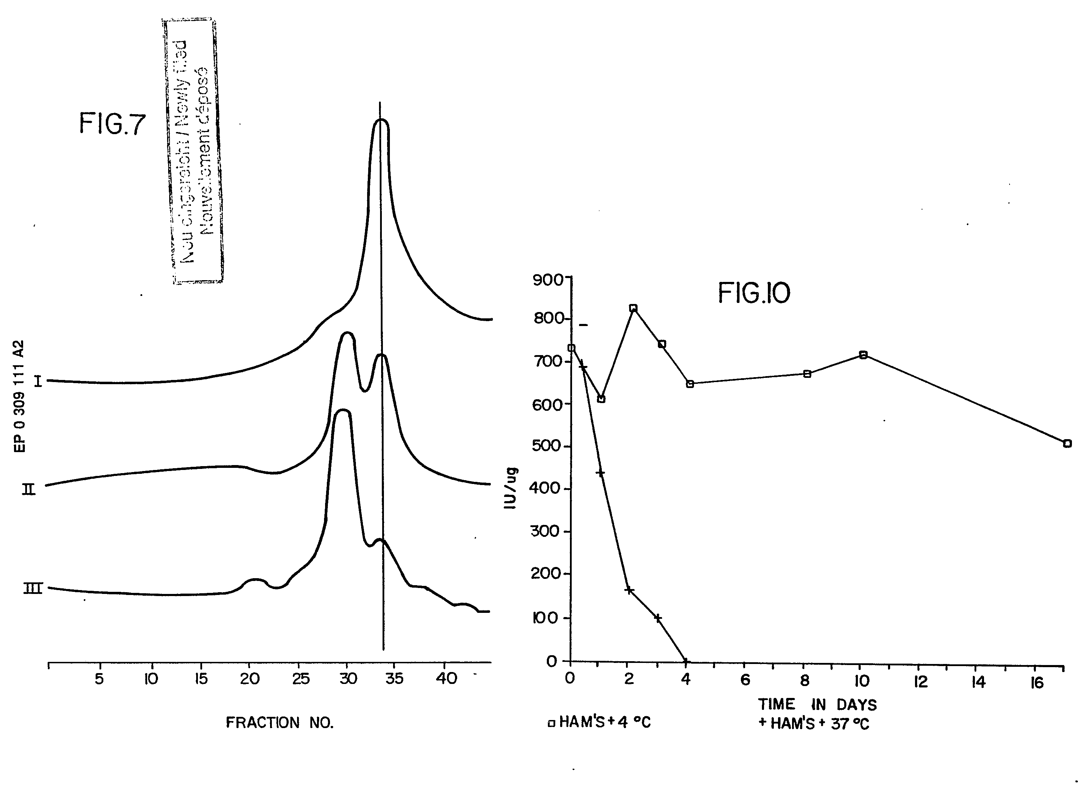

A comparison of the molecular weight species of tPA in the gel chromatography eluants of Cases I (perfusion), II (semi-batch) and III (batch) is shown in Figure 7. tPA is detected by electrophoresis on SDS gels, immunoassay, as well as by enzymic activity.

As seen in the figure, Case I yields predominantly a single peak corresponding to monomeric tPA, i.e., it migrates as a 70,000 dalton species. Case II yields two approximately equal peaks of tPA, one corresponding to the monomeric species (fractions 32-38), and a second migrating with an apparent molecular weight of approximately 110,000 daltons (fractions 27-30). Case III yields predominantly a species which migrates with an apparent molecular weight of approximately 110,000 daltons. In these cases the ratio of unaggregated (monomeric) tPA to aggregated tPA may be estimated by comparing the areas in peaks I and II, with the assumption that the peaks are symmetrical. A line is drawn through the center of peaks I for purposes of identification.

C.5. Characterization of the Species of tPA Obtained from Perfusion, Batch, and Semi-Batch Processes by Polyacrylamide Gel Electrophoresis Under Reducing Conditions

The fractions obtained by gel filtration chromatography as described in Sections C.3. and C.4. are analyzed by electrophoresis on polyacrylamide gels using the Laemmli procedure in the presence of a reducing agent. Under reducing conditions, the disulfide bonds linking the A and B chains as well as disulfide intermolecular bonds causing tPA aggregation are dissociated. However, the bonds linking the inhibitor protein(s) to tPA are not dissociated. Therefore, this technique allows an evaluation of tPA aggregates of the tpA-inhibitor species.

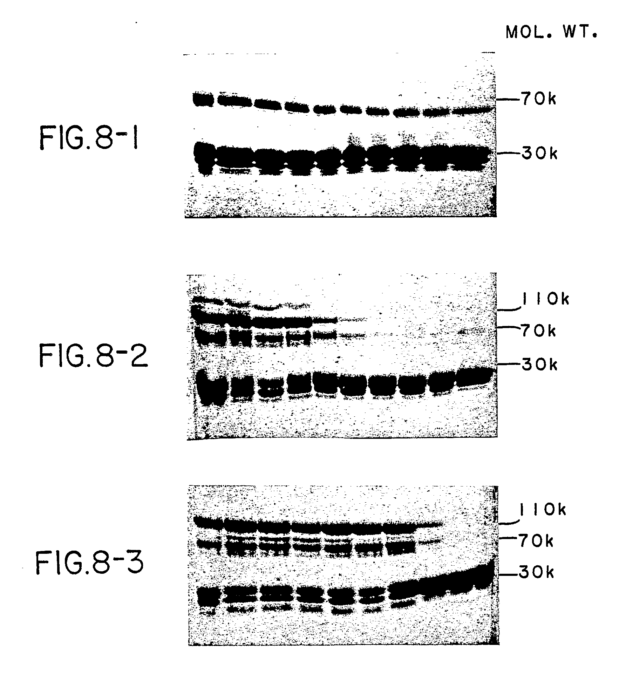

Fractions 27-35 from each of Cases I (perfusion), II (semi-batch), and III (batch) and of the material applied to the gel filtration columns are subjected to SDS polyacrylamide gel electrophoresis in the presence of a reducing agent, and the proteins visualized by protein stain. Reproductions of the stained gel photos are shown in Figure 8, where the extreme left hand lane is an aliquot of the sample applied to the column, and the subsequent lanes proceeding from left to right are from fractions 27-35, inclusive.

In case I, the material obtained from the perfusion effluent is relatively pure since the only bands visualized are primarily chains A and B of tPA migrating at approximately 30,000 daltons, and some of the uncleaved one chain tPA migrating at 70,000 daltons.

In comparison to case I, the material obtained from the batch and semi-batch processes, cases III and II, respectively, showed significant amounts of tPA migrating at approximately 110,000 daltons, in addition to the material migrating at 70,000 and 30,000 daltons. This 110,000 dalton material is believed to represent the tPA-inhibitor complex(s), discussed supra, which can copurify with tPA.

The identity of the protein staining material as tPA is verified by immunoblot assay of the gels using a monoclonal antibody directed against tPA.

C.6. Characterization by Polyacrylamide Gel Electrophoresis of tPA Which Elutes from Gel Filtration Chromatography

Electrophoresis on polyacrylamide gels under non-reducing and reducing conditions can be used to characterize the species of tPA present in the effluent. Under non-reducing conditions aggregates of tPA, which are comprised of tpA-inhibitor complexes and complexes formed from intermolecular cross-linking via sulfhydryl bonds, migrate with an apparent molecular weight of approximately 110,000 or greater, while unaggregated tPA migrates with an apparent molecular weight of approximately 70,000. Under reducing conditions, single chain (uncleaved) tPA migrates with an apparent molecular weight of 70,000, tPA-inhibitor complexes migrate with an apparent molecular weight of approximately 110,000, and dissociated A and B chains of tPA (resulting from the disruption of sulfhydryl bonds) migrate closely with an apparent molecular weight of approximately 30,000.

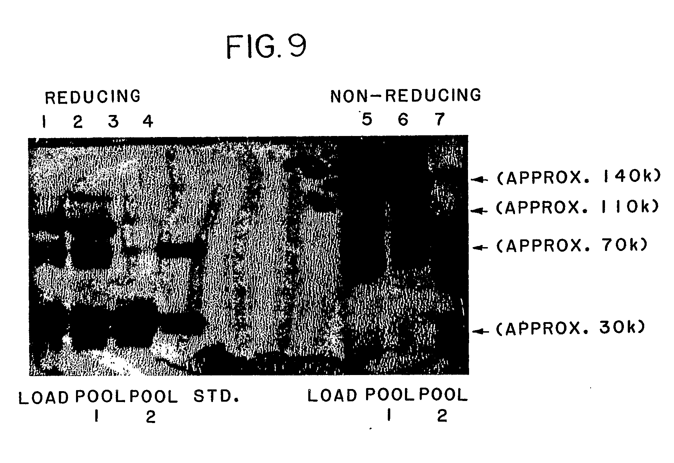

Material in each of the two peaks obtained from gel filtration chromatography of the tPA from the semi-batch process (Case II), as described in Section C.4., are formed into two separate pools, and the pooled material is subjected to SDS polyacrylamide gel electrophoresis under reducing and non-reducing conditions. Proteins are visualized by staining, and the presence of tPA in the bands is confirmed by immunoblot assay. A photograph of the stained gels is shown in Figure 9. The reduced samples are shown in lanes 1-4, and the non-reduced samples are shown in lanes 5-7. Lanes 1 and 5 are the proteins in the material loaded onto the gel filtration column. Lanes 2 and 6 are the tPA in the pooled fractions which migrate as approximately 110,000 dalton material. Lanes 3 and 7 are the tPA in the pooled fractions which migrate as approximately 70,000 dalton material. Lane 4 is a tPA standard.

As seen in the figure, the material in lane 7 (which is from peak 1 in Section C.4.) predominantly migrates as 70,000 molecular weight material under nonreducing conditions; and is transformed to two bands of approximately 30,000 daltons under reducing conditions. This appears to be relatively pure tPA isolated from the column, which accounts for approximately 1 3 - 1 2 of the material applied to the column.

The material in lane 6 (which is from peak 2 in Section C.4.) predominantly migrates as 110,000 or greater, molecular weight material under non-reducing conditions; material of approximate molecular weight of 70,000 daltons is also present. Under reducing conditions this material migrates as three separate species of tPA: the tpA-inhibitor complex with an apparent molecular weight of 110,000 daltons; single chain tPA with an apparent molecular weight of 70,000 daltons, and two chain tPA with an apparent molecular weight of 30,000 daltons. The conversion of the 110,000 or greater dalton species to the 70,000 dalton and 30,000 dalton species permits an estimate of the material which is present as aggregated tPA which results from sulfhydryl cross-linking, while the amount of material which remains as 110,000 dalton tPA permits an estimate of the material which is present as tpA-inhibitor complex.

The estimate is determined from densitometric scans of the polyacrylamide gels. Based upon the densitometry (not shown), the relative percentages of the species in the gel filtration peak of about 110,000 or greater dalton tPA were: 28% (110,000 dalton); 18% (70,000 dalton); and 54% (30,000 dalton, both bands). Since the 30,000 dalton tPA was absent under non-reducing conditions, it may be concluded that this species results from aggregation due to intermolecular sulfhydryl cross-linking. Therefore, the tpA-inhibitor complex represents approximately 28% of the tPA in this peak, while the sulfhydryl aggregated material represents approximately 54% of the material in this peak.

C.7. The Conversion of Unaggregated tPA to Aggregated tPA by Incubation in Conditioned Medium

The rate at which unaggregated tPA is converted to aggregated tPA under incubation conditions is determined by incubating unaggregated tPA in medium in which control cells which do not express tPA are grown, and determining the conversion of the unaggregated to the aggregated form.

Conditioned medium is obtained from control CHO cells which do not produce tPA by growing the cells in spinner culture to a PCV of 1, and harvesting the cells. The conditioned medium is "spiked" by the

12

addition of purified monomeric (unaggregated) tPA to a concentration of 30 micrograms of tPA per ml, a value which is expected in production conditions. The spiked medium is incubated at 37°C under cell propagation conditions. Samples are removed at zero time, at 18, 24, 48, 72, and 96 hours, and at 8, 10, 15, and 17 days, and the specific enzymic activities of the tPA in the samples is determined. In addition, aliquots of the samples are subjected to polyacrylamide gel electrophoresis under non-reducing conditions as well as reducing conditions, and the tPA containing bands are identified by immunoblot analysis. The species of tPA present as well as the amount in each species are determined from the immunoblots of the polyacrylamide gels.

The results of the incubation on enzymic activity are shown in table 3, where it may be seen that by 24 hours the specific activity of the tPA has decreased by approximately 30%. Continued incubation for 48 and 96 hours causes further decreases in enzymic activity, and by 96 hours the specific activity has decreased by almost 70%. Continued incubation to 17 days causes further decreases in enzyme activity. This decrease in activity is accompanied by an increasing amount of aggregated tPA, as shown by the appearance of tPA migrating with an apparent molecular weight of approximately 110,000 or greater daltons upon polyacrylamide gel electrophoresis under non-reducing conditions.

Table 3

| Inactivation of tPA | |
| --- | --- |
| Inc. Time (Hrs/Days) | Specific Activity (I.U./mg) |
| OT | 440,000 |
| 18 Hrs | 310,000 |
| 24 Hrs | 292,000 |
| 48 Hrs | 207,000 |
| 72 Hrs | 203,000 |
| 96 Hrs | 160,000 |
| 8 Days | 148,000 |
| 10 Days | 127,000 |
| 15 Days | 65,000 |
| 17 Days | 52,000 |

## C.8. The Effect of Serum on Aggregation of tPA

Perfusion techniques allow cells to be propagated in minimal amounts of serum. Utilizing the system described in C.2., tPA can be expressed by CHO cells in the absence of serum. Therefore, conditioned medium minus serum is used for the "spiking" of tPA. The tPA spiked medium is treated as described in Section C.7. to determine the aggregation of tPA caused by incubation in the serum-free conditioned medium

## C.9. Production of tPA in Batch and Semi-batch Processes and Collecting the Effluent at Desired Ratios of Unaggregated to Aggregated tPA

The cells used to express tPA and the cell culture conditions are as described in Section C.2. for the batch and semi-batch processes, except that the effluents are collected at a time when it has been calculated that the ratio of unaggregated to aggregated tPA will be at least 2:1. The calculation is based upon a spiking example as described in Section C.8., wherein the ratio of unaggregated to aggregated tPA is determined by the relative proportions of the 110,000 dalton species and 70,000 dalton species present in the effluent. The latter is determined by polyacrylamide gel electrophoresis under non-reducing conditions, followed by immunoblot analysis to determine the identity and amounts of the species. Alternatively, it may be determined by the decrease in specific enzymic activity of the added tPA.

C.10. Production of tPA in Perfusion Processes and Collecting the Effluent at Desired Ratios of Unaggregated to Aggregated tPA

The cells used to express tPA and the cell culture conditions are as described in Section C.2. for the
perfusion process, except that the effluents are collected at a time when it has been calculated that the ratio
of unaggregated to aggregated tPA will be at least 2:1. The calculation is determined as described in
section C.9. except that the conditioned medium is from CHO cell PCVs comparable to that expected in the
perfusion system, i.e. closer to PCVs of about 10 ml/L.

C.11. The Effect of Temperature on Preventing Aggregation of tPA in the Effluent

The rate at which unaggregated tPA is converted to aggregated tPA under at various temperatures is
determined by incubating unaggregated tPA in medium in which control cells which do not express tPA are
grown. and determining the conversion of the unaggregated to the aggregated form.

Conditioned medium spiked with tPA, as described in Section C.7., is incubated at temperatures
varying between 0° C and about 25° C. Samples are taken at zero time. 24, 36. 48, 72, and 96 hours to
determine the specific activity of tPA and the species of tPA present, as described in section C.7.
Appropriate controls include tPA incubated in buffer for the indicated times, and tPA which has been frozen
and freshly thawed.

C.12. The Effect of Incubation at 37° C Compared to Incubation at 4° C on Aggregation of tPA

The preventative effect of lowered temperature on the aggregation of tPA, as well as the effect of
removal of conditioned medium is determined by a "spiking" experiment as described in C.7., except that
the tPA is incubated in conditioned medium at 37° C and at 4° C. In addition, as a control, tPA which has
been frozen, and thawed is also examined.

Purified two chain tPA from recombinant CHO cells was spiked into two different media that had been
conditioned by the growth of a non-tPA producing CHO cell line. Cells were removed from the media, and
the tPA added back to a final concentration of 30 micrograms/ml, which represents optimal tPA expression
levels. The two media used were HAMS plus 5% fetal bovine serum (FBS) and a medium which was
serum-free (SFM). Media to which tPA had been added were maintained at 37° C for eight hours; the
samples were then split. and portions were maintained at either 4° C or 37° C for the remainder of the study
(17 days). Control samples were kept frozen at -40° C. Aliquots of tPA containing media maintained under
the two temperatures were withdrawn at various times. and the samples frozen at -40° C. All samples were
thawed and analyzed at the end of the experiment.

The samples were analyzed as follows.

1) S-2251 - Parabolic plasminogen activation assay. which measures the fibrin dependent tPA
activity.

2) S-2288 - Direct amidolytic activity of tPA, which measures only the activity of the serine protease
domain of the tPA.

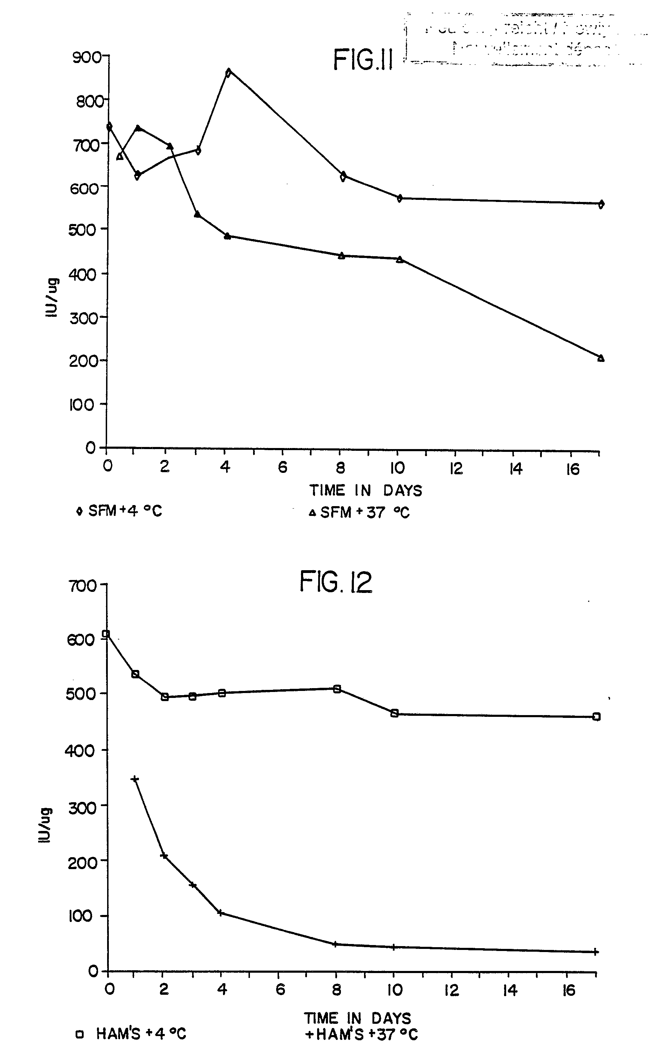

The results of the activity assays are presented in Figs. 10-13, which show the incubation in serum-
containing medium and serum-free medium, utilizing the fibrinogen activity assay (Figs. 10 and 11,
respectively), and the incubation in serum-containing medium and serum-free medium, utilizing the direct
amidolytic assay (Figs. 12 and 13, respectively).

It can be seen from the data in Figs. 10-13 that at 4° C, the tPA is remarkably stable in both media
throughout the 17 day study period. This result confirms that tPA retains full activity when transferred to a
4° C environment.

In contrast to the results obtained by transferring the tPA containing media to 4° C. the activity of the
samples maintained at 37° C was usually considerably diminished. However, the amount of activity lost was
dependent upon the presence or absence of FBS in the medium, as well as the activity assay used. The
loss of activity of tPA utilizing the direct amidolytic assay was not as pronounced as that utilizing the fibrin-
dependent assay, and in serum-free medium appeared to be negligible. Compare figures 1 and 2, with
figures 3 and 4. However, the fibrin dependent assay may be more reflective of the in vivo activity of tPA.
Utilizing this assay, in serum-free medium, approximately 70% of the initial tPA activity was lost by day 17 in samples which had been maintained at 37°C (Figure 11). These conditions are typical of batch culture processes. In serum-containing medium, fibrin-dependent tPA activity was not measurable after 4 days at 37°C (Figure 12).

C.13. Purification of Recombinant tPA Expressed in CHO Cells

A purification scheme used for CHO tPA is described below in steps 1-4. The flow rates used for the columns comprised of the materials used for separation were the manufacturer's recommended rates. The fractions containing tPA were monitored by determining the concentration of protein present, and the tPA present by an ELISA assay and/or a Western Blot assay and/or an enzymic assay for tPA activity.

The buffers referred to are as follows:

#1: 50 mM sodium acetate (pH 5.5), 100 mM sodium chloride;

#2: 50 mM sodium acetate (pH 5.5), 200 mM sodium chloride;

#3: 20 mM Tris chloride (pH 8.0), 1 M sodium chloride;

#4: 20 mM Tris chloride (pH 8.0), 1 M sodium chloride, 0.1 M arginine.

#5: 20 mM Tris chloride (pH 7.0), 1 M sodium chloride, 1 M arginine.

#6: 150 mM arginine-HCl, 50 mM sodium citrate (pH 6.0).

Step 1: A 5-liter S-Sepharose Fast-Flow bioprocess column (Pharmacia, 25.2 cm x 10 cm) was packed at approximately 400 ml/cm²·hr, equilibrated with five column volumes of Buffer 1 at approximately 300 ml/cm²·hr. Cell culture conditioned medium, which had been adjusted to pH 5.5 with dilute acetic acid, was loaded onto the column at approximately the same flow rate. The pH and conductivity of the media were such that the tPA bound selectively to the column by electrostatic forces, while the bulk of the serum proteins and non-proteinaceous material (i.e., nucleic acids, phospholipids, etc.) did not bind to the resin and were removed in the column flowthrough. The loaded column was washed with 3 column volumes of Buffer 2 at 300 ml/cm²·hr, and was then eluted with approximately 4 column volumes of buffer 3 at 30 ml/cm²·hr. The fractions containing tPA were pooled.

Step 2: A 4 liter bioprocess column (25.2 cm x 8 cm) of p-Aminobenzamidine (PABA), which was obtained from Pierce Chemical Co., was equilibrated with 5 column volumes of Buffer 3 at 100 ml/cm²·hr, and loaded with the pooled fractions containing tPA from Step 1. The loaded column was washed with 3 column volumes of Buffer 4, using the same flow rate, and eluted with Buffer 5 at 10-20 ml/cm²/hr. The fractions containing tPA were pooled.

Step 3: The pooled fractions from Step 2 were diluted with 2 volumes of Tris chloride, pH 8.0, and precipitated by the addition of solid ammonium sulfate, 300 grams solid/liter of diluted eluant. The material was stirred until the ammonium sulfate was fully dissolved, and was maintained at 4°C for a minimum of 4 hours. The precipitate was collected by centrifugation (5000 rpm for approximately 30 minutes). The pellets were stored at -70°C until gel filtration chromatography.

Step 4. The ammonium sulfate pellets from Step 3 were thawed at 4°C and resolubilized in a restricted volume of Buffer 6 to yield a tPA concentration of about 15-20 mg/ml. The resulting tPA solution was subjected to gel filtration through a column of Sephadex G-25 (Pharmacia), which had been pre-equilibrated against Buffer 6. Elution was with the same buffer, at the manufacturer's recommended flow rates. The tPA is excluded from the gel, and hence was collected in the gel exclusion volume.

Industrial Applicability

The processes of the invention are useful for increasing the yields and quality of commercially produced tPA which is synthesized in cell culture systems. It is expected that highly purified tPA, which is in the advanced stages of clinical trials, will soon be required in multi-kilogram quantities, and that the commercial source of this product will be cell culture systems. The product from these systems, to be useful for pharmaceutical purposes, must possess a high degree of purity and activity, and therefore must be free of high molecular weight aggregates that can reduce activity and induce harmful immunogenic side effects, particularly in humans. The processes of the invention substantially decrease the amount of aggregated tPA produced in cell culture systems, thereby allowing increased yields of tPA which is acceptable for pharmaceutical purposes. In addition, the reduction in the aggregated form should significantly increase the ease with which the desired form of tPA can be purified, thereby decreasing the cost of production.

The tPA produced by the processes of the invention has, after purification, significant commercial utility as a pharmaceutical product for the relief of disorders related to blood clots. In addition, the purified products of the invention have commercial utility as a standard, which may be used to monitor the commercial processes used for the invention, and which may be used in the diagnosis of disorders related to elevated or lowered levels of tPA.

**Claims**

1. A process for obtaining higher yields of unaggregated tissue plasminogen activator (tPA) from tPA expressing cells in culture comprising:

a) removing tPA containing effluent from a cell culture chamber containing tPA expressing cells while substantially more unaggregated tPA is present than aggregated tPA; and

b) subjecting the removed tPA containing effluent to a condition which substantially prevents said unaggregated tPA from aggregating.

2. The process of claim 1, wherein the tPA expressing cells are mammalian cells.

3. The process of claim 2 wherein the tPA expressing cells are mammalian cells expressing tPA encoded within a recombinant DNA expression vector.

4. The process of any one of the preceding claims wherein the removing of the effluent occurs when active unaggregated tPA and inactive aggregated tPA are in the effluent in a ratio of at least 2:1, preferably of at least 3:1, and more preferably of at least 4:1.

5. The process of any one of the preceding claims wherein the removed effluent is rapidly chilled to a temperature in which the aggregation of active tPA is substantially prevented, wherein the temperature is preferably in the range of about $0^{\circ}$ C to about $15^{\circ}$ C, and is more preferably in the range of about $1^{\circ}$ C to about $10^{\circ}$ C.

6. A process for obtaining higher yields of unaggregated tPA from cells in culture comprising:

a) propagating cells capable of producing and secreting tPA in a perfusion system under conditions which allow the cells to synthesize tPA;

b) removing tPA containing effluent from the perfusion system while substantially more unaggregated tPA is present than aggregated tPA; and

c) subjecting the removed tPA containing effluent to a condition which substantially prevents said active unaggregated tPA from aggregating.

7. The process of claim 6 wherein the cells are CHO cells transformed with an expression system encoding tPA, and wherein the removed effluent is rapidly chilled to a temperature in which the aggregation of active tPA is substantially prevented, wherein the temperature is preferably in the range of about $0^{\circ}$ C to about $15^{\circ}$ C, and is more preferably in the range of about $1^{\circ}$ C to about $10^{\circ}$ C.

8. The process of any one of the preceding claims, wherein the removed effluent is rapidly subjected to a procedure which isolates the tPA from the conditioned cell culture medium.

9. A process for obtaining higher yields of unaggregated tissue plasminogen activator (tPA) from tPA expressing cells in culture comprising:

a) continuously removing conditioned media from cells producing tPA to a low temperature environment of about $4^{\circ}$ C;

b) adjusting the pH and ionic strength of the medium to conditions wherein tPA is selectively bound to a cation exchange support;

c) treating the adjusted medium with a cation exchange support so that tPA is selectively adsorbed;

d) eluting the adsorbed tPA from the cation exchange support;

e) adjusting the pH and ionic strength of the tPA containing eluate to conditions wherein tPA is selectively adsorbed to an affinity support;

f) treating the adjusted eluate of e) with an affinity support to selectively adsorb the tPA;

g) eluting the selectively adsorbed tPA from the affinity support;

h) causing the tPA in the tPA containing eluate of g) to precipitate by the addition of salt, and capturing the tPA containing precipitate;

i) dissolving the tPA containing precipitate of h) in a solution containing arginine HCl and sodium citrate, wherein the arginine HCl is preferably about 150 mM and the sodium citrate is preferably about 50 mM, and the pH is preferably about 6;

16

j) treating the tPA containing solution of i) with a gel filtration support equilibrated against the solution of i); and

k) eluting the tPA from the gel filtration support and recovering the tPA containing fraction.

10. A pharmaceutical composition comprising a tPA obtained by the process of any one of the preceding claims together with a pharmaceutically acceptable diluent.

HEAT EXCHANGE
SYSTEM

LEVEL
PROBE

pH
PROBE

MEDIUM
RESERVOIR

PRODUCT
RESERVOIR

HARVEST
VESSEL

FILTRATION
SYSTEM

LIQUID
TRANSFER
SYSTEM

GROWTH VESSEL

BASE
RESERVOIR

◄4°C        37°C►

FIG.I

HEAT EXCHANGE
SYSTEM

SETTLING
BOTTLE

LEVEL
PROBE

pH
PROBE

LIQUID
TRANSFER
SYSTEM

MEDIUM
RESERVOIR

PRODUCT
RESERVOIR

FILTRATION
SYSTEM

MICROCARRIERS

GROWTH
VESSEL

BASE
RESERVOIR

◄4°C        37°C►

FIG.2

HEAT EXCHANGE
SYSTEM

$O_2$

MEDIUM
RESERVOIR

PRODUCT
RESERVOIR

GAS PERMEABLE
TUBING
DISTRIBUTED LUNG

$CO_2$

◀ 4°C    37°C ▶

## FIG.3

IU/ug

TIME IN HOURS

◇SFM +4 °C    △SFM +37 °C

## FIG.13

FIG.4

PERFUSION

PACKED CELL VOLUME (ml/L)

COLLECTION PERIOD

DAYS IN CULTURE

FIG.5

SEMI BATCH

PACKED CELL VOLUME (ml/L)

DAYS IN CULTURE

EP 0 309 111 A2

FIG.6

FIG.7

Neu eingereicht / Newly filed
Nouvellement déposé

EP 0 309 111 A2

I

II

III

FRACTION NO.

FIG.10

900
800
700
600
500
400
300
200
100
0

IU/ug

0   2   4   6   8   10   12   14   16

TIME IN DAYS

□ HAM'S + 4 °C
+ HAM'S + 37 °C

MOL. WT.

FIG.8-1

—70k

—30k

FIG.8-2

—110k

—70k

—30k

FIG.8-3

—110k

—70k

—30k

FIG.9

REDUCING

1  2  3  4

NON-REDUCING

5  6  7

LOAD POOL POOL  STD.

1     2

LOAD POOL POOL

1     2

← (APPROX. 140k)
← (APPROX. 110k)
← (APPROX. 70k)

← (APPROX. 30k)

# FIG.II

900
800
700
600
500
400
300
200
100
0

IU/ug

0  2  4  6  8  10  12  14  16

TIME IN DAYS

◊ SFM+4 °C          ▵ SFM + 37 °C

# FIG. 12

700
600
500
400
300
200
100
0

IU/ug

0  2  4  6  8  10  12  14  16

TIME IN DAYS

□ HAM'S +4 °C       + HAM'S +37 °C